# EUROPEAN PATENT SPECIFICATION

(11) **EP 2 347 717 B1**
(45) Date of publication and mention of the grant of the patent: **16.01.2013**
(21) Application number: 11150958.4
(22) Date of filing: 24.08.2007
(51) Int. Cl.: A61B 8/08, A61B 17/34, A61B 19/08, A61B 19/02

(54) **Needle guide for an imaging device**
Nadelführung für eine Bildgebungsvorrichtung
Guide d'aiguille pour dispositif d'imagerie

(30) Priority: 24.08.2006 WO PCT/US2006/033145; 02.03.2007 US 712907; 06.06.2007 US 689717
(43) Date of publication of application: 27.07.2011
(62) Divisional of application: 07811550.8
(73) Proprietor: ULTRASOUND VENTURES, LLC, Durham NC 27707 (US)
(72) Inventor: Park, Robert, Durham, NC 27707 (US); Mosler, Theodore J., Raleigh NC 27614 (US); Jarnagin, Scott P., Seattle WA 98109 (US)
(74) Representative: Israelsson, Stefan

(56) References cited:
- WO-A1-96/32066
- US-A1- 2005 131 291
- US-B1- 6 296 614
- US-B1- 7 087 024

## Description

### FIELD

The disclosure relates generally to imaging devices, such as an ultrasound device, and more particularly a needle guide.

### BACKGROUND

In a typical ultrasound guided procedure, a doctor will place a small, handheld probe known as a transducer on a patient's skin. The transducer converts electrical energy to acoustic energy. Acoustical energy is transmitted from the transducer and into the patient's body in the form of sound waves. The transmitted sound waves are either reflected back towards the transducer or absorbed by the medium, depending on the acoustical impedance. The reflected sound waves are converted into electrical signals which are used to form a real time two-dimensional image of a portion of the patient's body. This ultrasound image may be used to assist a health professional with locating a point where an invasive medical device, e.g., a needle, is inserted. After locating the correct insertion point, the health professional may then begin the medical procedure, such as insertion of a catheter, administration of a local anesthetic, or removal of tissue as in a biopsy.

Before beginning a procedure, it is necessary to cover the ultrasound device to assure that sterility is maintained during the procedure. Typically, a sterile sleeve or condom made of a flexible, sterilizable material is draped over the device to form a sterility barrier. A sterility barrier is intended to refer to a seal, bond, covering, etc. that is effective in preventing micro-organisms or other contaminates from migrating from within the sleeve, condom, etc., to the exterior, sterile environment. An acoustic coupling gel is placed in the sleeve before placing the transducer in the sleeve to ensure consistent contact between the sound transmitting/receiving end or head of the transducer and the sleeve. This contact is necessary to ensure there are no artifacts in the ultrasound image due to the presence of air pockets between the head and sleeve. The coupling gel may be applied to the sleeve at the time of the procedure or the gel may be pre-applied to the sleeve. In the later case, a breakable seal is usually included in the sleeve to protect the gel from contaminates. This type of cover is described in U.S. Pat. No. 5,676,159. The end opposite the head of the transducer is typically sealed using a rubber band wrapped around the sleeve.

Brackets are sometimes provided with an ultrasound device for purposes of mounting a needle guide. The needle guide is used to facilitate a longitudinal or transverse type ultrasound guided needle insertion procedure. In the "transverse" type, the guide is arranged so that the needle is inserted into the patient along a plane transverse to the ultrasound image plane. In the "longitudinal" type, the needle is inserted into the patient in a plane parallel to the ultrasound image plane.

One known ultrasound device for assisting a health professional with needle placement in a body is the ilook™ personal imaging tool, sold by SonoSite ®, Inc., which includes a series of removable needle guides and a bracket for mounting the needle guides to the transducer. The device is used to place a needle at a target beneath the skinline by real-time visual identification of the target via an ultrasonic image. The needle guide is arranged on the transducer so that a needle received therein will extend approximately perpendicular to the sonic scanning plane. Thus, the SonoSite ®, Inc. device is a transverse-type device. A sterile needle guide is snap-fit on the bracket. There is more than one-type of needle guide to choose from. The choice depends upon the distance between the skinline and the needle's target in the body. The needle guide has a door that can be locked in a closed position by a slidable switch, thereby retaining the needle shaft between the door and a semi-circular recessed area. The needle is placed in this recessed area and the door is closed to hold the needle therein. The transducer with needle is then placed on the skinline and the top of the vessel is located via the sonic image. The needle is then inserted into the body. After the needle has reached the target, the transducer is removed from the needle, which requires a manual unlatching of the door of the needle guide. A related needle guide is described in U.S. Publication No. 2005/0131291.

WO 96/32066 A1 describes a needle support assembly comprising a block having a V-shaped channel for receiving a needle. A locking arm includes a ridge disposed to engage the needle when in a closed position. The locking arm includes a latch portion including serrations that inter-engage serrations on the block.

### SUMMARY

The present invention provides a needle guide as defined in claim 1 or claim 9. Further embodiments of a needle guide is defined in the dependent claims.

In addition to a description of a needle guide, the present disclosure also describe other parts with which a needle guide according to the invention can be used. Also methods in which a needle guide may be used will be described.

The disclosure is directed to a sterile cover and needle guide for an ultrasound probe. The sterile cover may include a sterile shell and sheath sealed to an opening of the shell. The ultrasonic probe is received in the shell and then covered by the sheath, thereby sterilizing the probe. The sterile cover may be adapted for use with a probe that is used to insert a medical device, such as a needle, into a body using an ultrasonic image generated by the probe and displayed on a nearby monitor. For example, the shell may include structure for attaching a needle guide to a needle positioning and detecting device provided with the probe. Alternatively, the shell may include an exterior mount or bracket for attaching the needle guide to the shell.

In one aspect, an ultrasound probe is enclosed within a sterile cover that includes an opening for receiving a needle clip on the probe. The needle clip is inserted into the opening and connected to a shaft provided with the probe. The needle may be rotated through a continuum of angles for purposes of locating a desired entry point for a needle received on the clip. The opening and/or clip may include structure that formes a sterility barrier between the clip and the opening. According to this aspect of the disclosure, the probe, sterile cover and clip may correspond to a transverse-type ultrasound guidance system.

In another aspect, a kit for positioning a needle during a medical procedure includes a sterile needle guide including a connector and a first sealing part provided with the connector, a sterile shell having an interior attachable to an ultrasound probe, an opening to the shell interior configured to receive the connector, and a second sealing part provided with the opening. The first and second sealing parts cooperate to form a sterility barrier between the shell and the connector when the connector is received in the opening. The first sealing part may be an o-ring received in an annular groove and the second sealing part may be a cylindrical surface for receiving the first sealing part. In this aspect of the disclosure, the needle guide, when received on the shell, may be constructed so that it is both rotatable about the shell and configurable in a position for retaining a needle received in the guide when finger pressure is applied to the guide. The kit may be configured for use with a transverse-type or longitudinal-type ultrasound guidance system.

In another aspect, a sterile cover includes a shell having an acoustic window and an opening for receiving an ultrasound probe within the shell, and a sheath having an edge that is sealed to the opening of the shell and configured to cover the probe. When a probe is received in the sterile cover according to one aspect of this disclosure, the probe is made sterile and ready for use. The shell may also be constructed to mount a needle guide to the shell exterior or provide an opening for securing a needle guide to a positioning and detecting device associated with the probe. In the later case, the probe is made sterile when received in the sterile cover and the clip is connected to the positioning and detecting device.

In another aspect, a method for sterilizing an ultrasound probe includes the steps of providing a sterile cover including a shell and a sheath sealed to an end of the shell and arranged to form a passage for receiving a head of the probe in the shell, inserting the head into the passage, connecting the head to the shell and sliding the sheath over the probe.

According to another aspect of the disclosure, a needle guide is operated by applying finger pressure to the guide so as to retain a needle shaft in the guide. When finger pressure is applied to the guide, the guide forms a closed space for the needle shaft. The needle is kept with the guide while finger pressure is applied to the guide. In this manner, the guide may be separated from the needle shaft by relieving the finger pressure with little if any unintended movement of the needle shaft while the needle is embedded in a patient.

In one aspect, a needle clip includes a holder forming a cradle for a needle shaft and a cover. The cover is moveable between a first position adjacent the cradle to a second position covering the cradle. A flexible member is connected to the cover and the holder. When the cover is in the second position, the cover and cradle form a closed space adapted for retaining the needle shaft in the cradle, the member is elastically deformed and the cover is devoid of a mechanical engagement with the holder. The flexible member may be a beam or a spring and the cover may be cantilevered from a finger rest, which may connect the flexible member to the cover.

In another aspect, a needle guide includes a holder forming a surface including a cradle adapted to receive a needle shaft and a ledge disposed above and at a first side of the cradle, and a cover having an end. The cover is moveable between a first position located at a second side of the cradle and a second position. When the cover is in the second position, the cover is disposed over the cradle and the end is between the surface and the ledge, the cover and cradle form a closed space adapted for retaining the needle shaft in the cradle and the cover is devoid of a mechanical engagement with the holder. The ledge may be formed by a notch, a U-shaped or C-shaped member formed on the holder. The ledge can function as a surface that restrains separation of the cover from the cradle, e.g., if the needle shaft presses against the cover.

The needle clip may be used with a shell that receives an ultrasound probe. The shell may include structure for attaching the clip to the exterior of the shell, or receiving a connector of the needle clip in an opening of the shell and attaching the connector to a shaft provided with the probe. In the later case, the needle clip may include a sealing member which, when engaged with the opening in the shell forms a sterility barrier with the shell.

The needle guide may be used with a transverse-type or longitudinal-type ultrasound imaging device, and an imaging device that provides a fixed mount and/or adjustable mount for the needle guide.

In another aspect, a method for inserting a needle into a body includes the steps of providing a needle guide on an ultrasound probe, applying finger pressure to the needle guide, wherein the applied finger pressure forms a closed space for retaining a needle shaft in the guide, locating a needle insertion point on the body, inserting the needle shaft into the closed space, inserting the needle into the body, and after the needle is inserted into the body, removing the applied finger pressure so that the needle guide can be separated from the needle shaft.

.These and other aspects of the disclosure will become apparent to those skilled in the art after a reading of the following description when considered with the drawings.

### BRIEF DESCRIPTION OF THE DRAWINGS

FIG. 1 is an exploded perspective view of an ultrasonic probe and sterile kit for the probe.

FIG. 2 is a perspective view of the probe of FIG. 1.

FIG. 3 is a perspective view of a sterile cover of the kit of FIG. 1.

FIG. 4 is a perspective view of the sterile cover of FIG. 3 with a head of the probe received in a shell of the sterile cover.

FIG. 5 is a perspective view of the sterile cover and probe of FIG. 3 with a sheath of the sterile cover covering the probe.

FIG. 6 is a partial side cross-sectional view of the probe with the head connected to the shell.

FIGS. 7 and 8 are side and perspective views, respectively of a needle clip of the kit of FIG. 1.

FIG. 9 is a partial perspective view of the sterile cover and probe of FIG. 5 with the needle clip of FIG. 7 connected to the shell.

FIG. 10 is a partial top cross-sectional view of the needle clip, sterile cover and probe of FIG. 9.

FIG. 11 is a side view of the needle clip of FIG. 7 with finger pressure applied to the needle clip.

FIG. 12 is a perspective view of a shaft of a needle positioned for insertion into the needle clip.

FIG. 13 is a perspective view of the needle shaft of FIG. 12 retained in the needle clip.

FIG. 14 is a perspective view of the needle shaft of FIG. 12 when embedded in a patient.

FIG. 15 is a perspective view of the needle clip after it has been separated from the embedded needle of FIG. 14.

### DETAILED DESCRIPTION OF THE DISCLOSED EMBODIMENTS

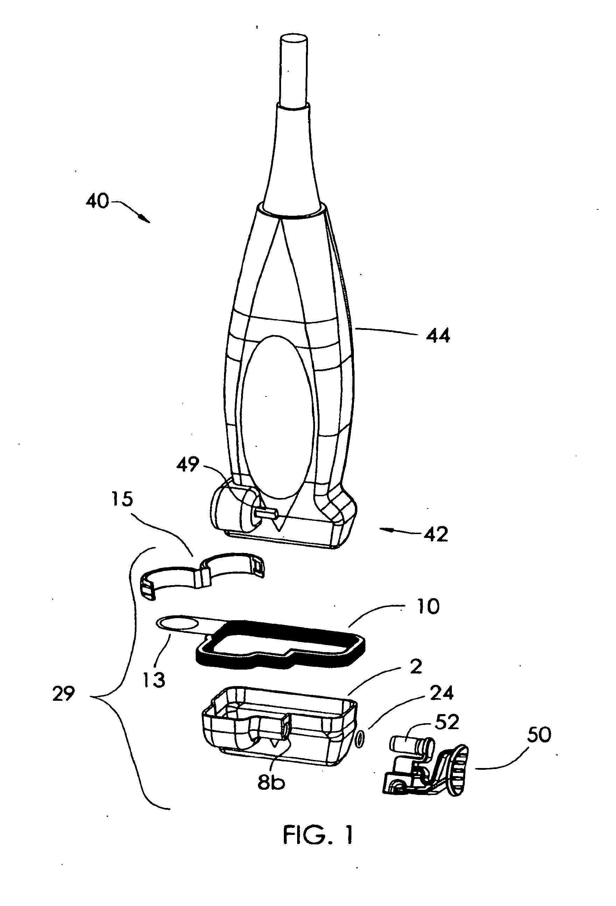

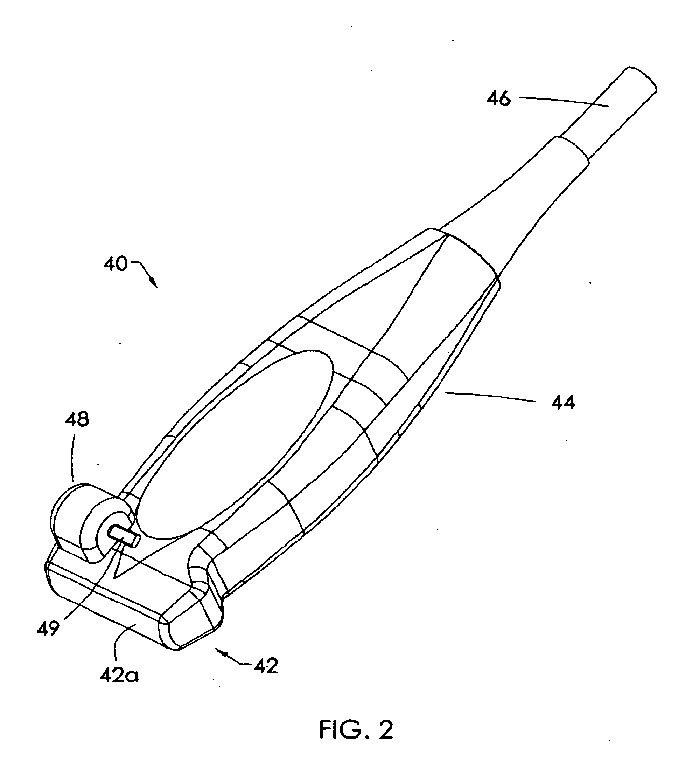

FIG. 1 is an exploded perspective view of an ultrasound guidance device according to one aspect of the disclosure. This device includes a sterile kit 29 for use with an ultrasonic probe 40 as in, for example, the ultrasonic probe described in U.S. Application Serial No. 11/508,300. With reference to FIG. 2, the probe 40 includes a body 44 containing the probe's transducer, a transmitting/receiving end or head 42 which emits and receives ultrasonic signals, a cord 46 for sending ultrasonic images to a nearby monitor (not shown), and a needle positioning and detecting device. The needle and positioning device includes a position encoder (or potentiometer) coupled to a rotatable shaft 49. The position encoder is contained in the portion 48 of the probe body 44 and the shaft 49 protrudes out from this housing 48 so that a needle guide may be received on the shaft 49. The shaft 49 may be sealed via o-rings or a similar sealing method to the housing 48. This can prevent contaminates from entering the housing 48 and thereby possibly interfering with the proper functioning of the needle positioning and detection device. The needle positioning and detector device enables a health professional to position or reposition a needle held on the probe 40 (via a needle clip received on the shaft 49), rotate that needle through a continuum of angles and track its position relative to a needle target displayed on a nearby monitor.

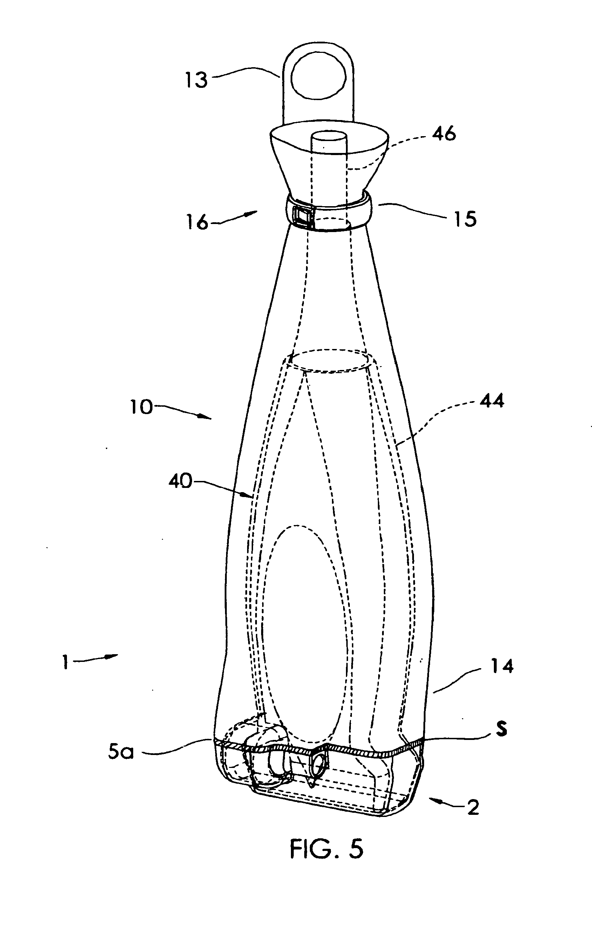

With reference to FIG. 1, the kit 29 includes a sterile cover 1 and a disposable needle clip 50. The sterile cover 1 includes a shell 2 and a sheath 10 secured to the shell 2. When fully assembled, the head 42 of the probe 40 is placed in the shell 2 and the sheath 10 is pulled or slid over the body 44 and secured thereto by a fastener 15 which may be included as part of the kit 29, see FIG. 5. A connector 52 of the needle clip 50 is then inserted into an opening 8b formed in the shell 2 and connected to the shaft 49 of the needle positioning and detecting device.

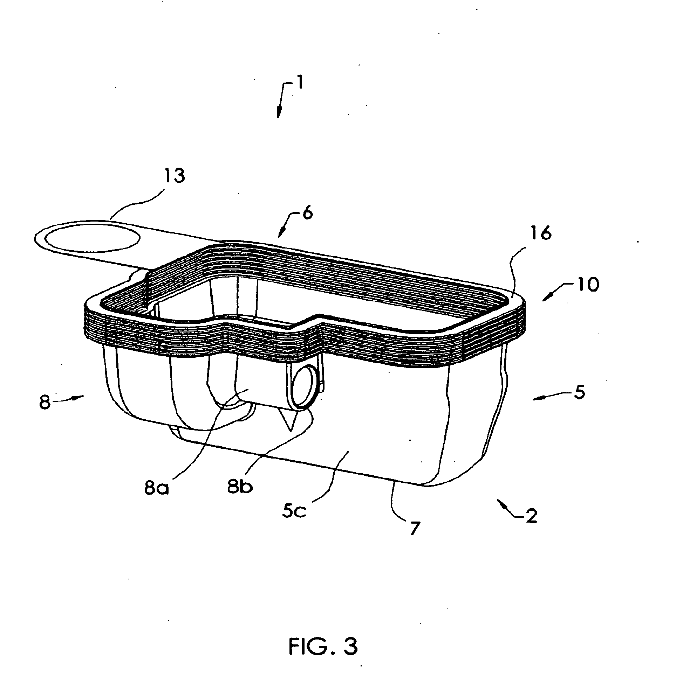

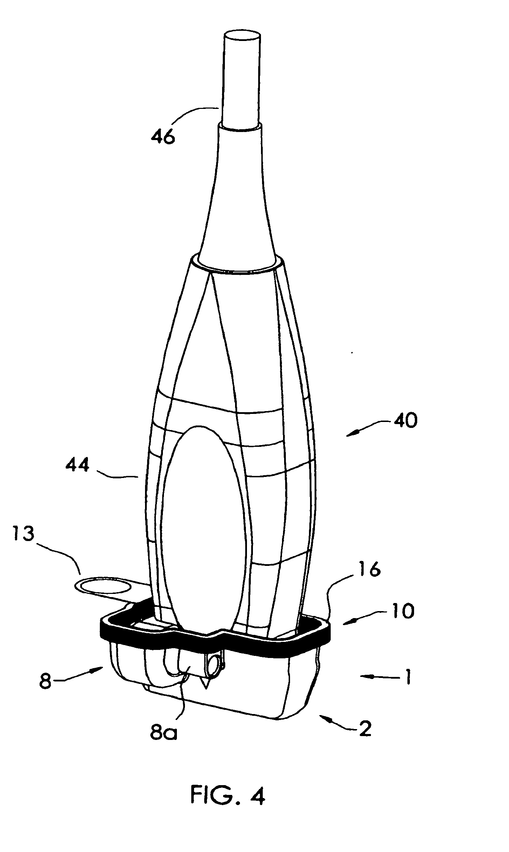

With reference to FIG. 3, the sterile cover 1 is shown in a stowed or folded configuration. The probe 40 is received in the shell 2, FIG. 4, and then the sheath 10 is pulled over the probe body 44, FIG. 5, and secured by the fastener 15. According to a first disclosure, the sterile cover 1 is sized to receive the probe 40 and hence includes accommodations for the housing 48 of the body 44. The sterile cover 1 may alternatively be formed to enclose various shaped transducer bodies and/or heads, with or without a needle bracket or needle positioning device without departing from the scope of this disclosure.

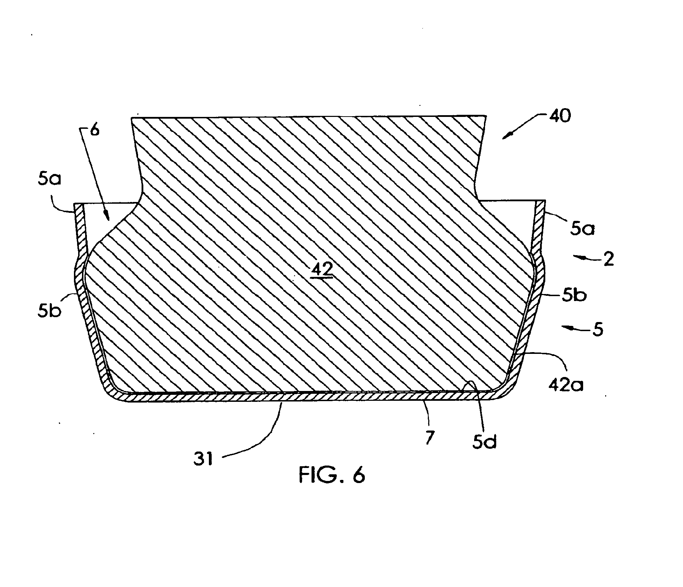

With reference to FIGS. 1 and 3-6, the shell 2 is preferably formed as a one-piece injection molded part. The head 42 of the probe 40 is received in an interior space 6 of the shell 2. This interior space 6 is formed by an acoustic window 7 and walls 5 which surround the window 7. The walls 5 define an opening 5a at an upper end and tapered portions 5b at opposed sides which extend from the opening 5a to the window 7 and conform to tapered surfaces 42a of the head 42, see FIG. 6. The tapered portions 5b are preferably shaped to provide a snap-fit engagement with the head 42, although the shell 2 may also be formed so that a frictional or elastic press-fit with the head 42 is made. In either approach, the shell - head engagement can be sufficient to ensure that the head 42 is held in place during the ultrasound procedure. This ensures there are no air pockets between the interior surface 5d of the shell 2 and the head surface 42a, which can cause artifacts to appear in the ultrasonic image. When inserted, the head 42 is put in contact with an acoustic coupling gel lining the interior surface 5d of the shell 2. The gel may pre-applied to the interior surface 5d. In this case, a removable lid stock may be applied to the shell opening 5a to protect the gel from contaminates. The gel may also be applied to the surface 5d just prior to inserting the head 42 into the shell 2. The shell 2 may include finger-pressure actuated release areas so that the head 42 may be easily removed from the shell 2. For example, one or more of the walls 5 may include a bulge which, when depressed cause the tapered portions 5b to elastically deflect away from the surfaces 42a of the head 42, thereby releasing the head 42 from the shell 2.

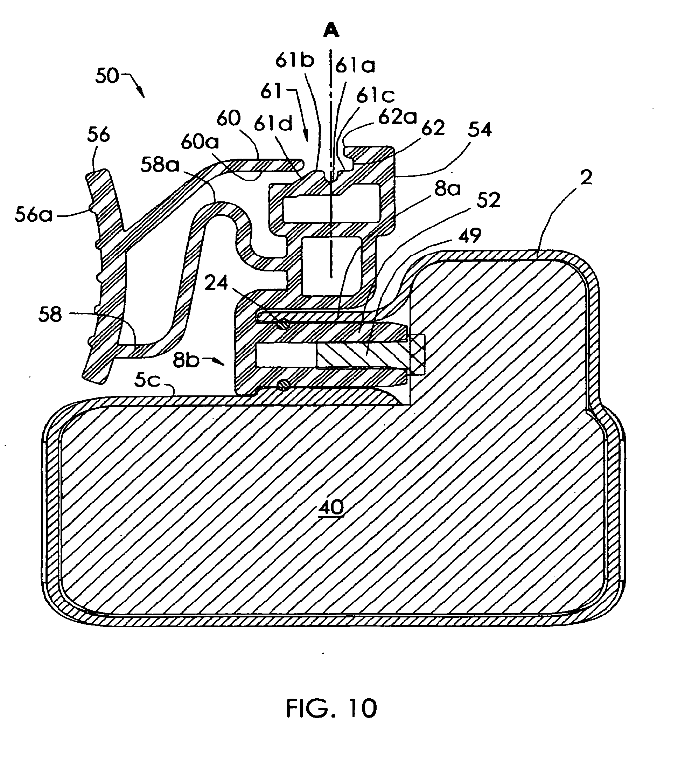

A front wall 5c of the shell 2 may have a section formed to accommodate the housing 48 of the probe's positioning and detecting device. This section includes an extension 8 forming a passage 8a for the needle clip connector 52 and shaft 49, and an opening 8b for receiving the connector 52, see FIG. 10. The opening 8b may include a flexible edge which, when engaged with the clip 50, produces an audible snap fit between an outer sleeve of the clip 50 and walls of the passage 8a so that a user is given an audible confirmation that a tight seal or sterility barrier has been achieved between the connector 52 and the walls of the passage 8a. The sterility barrier may be achieved by over-molding or fitting an o-ring against a cylindrical surface formed in the passage 8a or on the connector 52, or an integrated seal area may be formed in the opening 8b. Alternatively, a sterility barrier may be formed by engaging a cylindrical surface formed in the opening 8b with an annular ridge formed on the connector 52. Preferably, an o-ring 24 is placed on the connector 52, see FIG. 10. The o-ring or sealing structure could be made from any number of elastomeric materials, such as natural rubbers and latex, silicones, polyisoprenes, etc, and may be a purchased component. The o-ring or sealing structure may also be used to provide a desired degree of frictional resistance to rotational movement of the clip 50 during a procedure, in effect functioning as a detent. This may be desirable is it permits a health professional to maintain an angular position of the clip 50 and/or to facilitate precise angular adjustments of the needle clip 50 during a procedure. Since the interfering passage 8a and connector 52 components are intended as single-use only, a desired resistance can be reliably maintained over the life of the product. A detent may alternatively be incorporated into the positioning and detecting device.

The shell 2 may be formed using various semi-rigid polymers such as polypropylene, polyethylene, S-B copolymer, PC-ABS or the like. The material may be selected in order to achieve a desired clarity for the ultrasound. The undercut features of the shell 2 may be popped off of the mold, particularly if the plastic is a softer material, such as a polypropylene or polyethylene.

A second component of the illustrated sterile cover 1, the sheath 10, will now be discussed. With reference again to FIGS. 1 and 3-5, the sheath 10 is connected to the shell 2 and adapted for being slid over the body 44 of the probe 40 after the probe 40 has been connected to the shell 2, as depicted in FIG. 5. An upper edge 16 of the sheath may include a pull tab 13 that is used to pull the sheath 10 over the probe body 44. The sheath 10 may be formed from a thin, extruded material, such as a polyethylene, polyurethane, or similar film material, which can be pre-folded, preferably in an accordion-like fashion. The sheath may also be dip molded. The sheath may be considered as the flexible part of the sterile cover 1 and the shell 2 the rigid part.

Referring to FIG. 5, which shows the probe 40 enclosed within the sterile cover 1, a bottom edge 14 of the sheath 10 is sealed to the shell 2 by a seal s along the entire perimeter of the opening 5a. The upper edge 16 of the sheath 10 extends up to the cord 46 and is secured thereto by the fastener 15, e.g., a C-clip (a fastener may be secured to the sheath 10 so that it may be used to both pull the sheath 10 over the probe and secure the sheath 10 to the cord 46). The seal s may be formed by solvent bonding of the edge 14 to the opening 5a. A heat seal, sonic or RF weld or other methods may be used. These connection methods may serve not only the purpose of securely retaining the sheath 10 to the shell 2, but also forming a consistent seal along the entire perimeter of the opening 5 to ensure that sterility is maintained. An effective connection and sterility barrier may also be provided by a seal formed from a press fit between the opening 5a and the lower edge 14. In such a case, the opening 5a may include along its perimeter a channel or groove that receives, via a press-fit, a flexible rim disposed along the perimeter of the lower edge 14 of the sheath 10. With reference again to FIG. 5, when the sheath 10 is fully deployed and the fastener 15 secured as shown, the only passageway between the interior of the sterile cover 1 and the external environment is at the opening 8b, which receives the clip connector 52.

A procedure for assembling the ultrasonic device of FIG. 1 will now be discussed. The kit 29, containing the sterile cover 1 and one or more needle clips 50, is removed from a sterile packaging. The acoustic coupling gel may be provided with the kit 29 and applied directly to the interior 6 of the shell 2. Preferably, the gel is already present within the shell and protected from contaminates by a hermetic seal, e.g., a lid stock covering the opening 5a of the shell 2. The lid stock is removed from the shell and the head 42 of the probe 40 snap-fit into the shell 2, thereby forming a consistent acoustic medium between the face 42a of the head 42 and the window 7 of the shell 2 for sound transmission (FIG. 6). The pull tab 13 is then used to slide the sheath 10 over the body 44 of the probe and the fastener 15 secures the sheath 10 to the cord 46. The connector 52 of the sterile clip 50 is then inserted into the opening 8b of the shell 2. At this point, the ultrasonic device is sterile and ready for use.

The embodiments of the sterile cover 1 thus described are preferably intended for use with an ultrasonic probe equipped with an adjustable needle guide feature, e.g., the ultrasonic probe described in U.S. Application Serial No. 11/508,300. However, it will be understood from the foregoing that other embodiments of the sterile cover 1 are within the scope of this disclosure. The sterile cover 1 may be constructed for use with a probe that uses a longitudinal-type needle positioning and guidance device or a probe that does not use a needle guide. In these cases, the shell 2 and sheath 10 would be formed with an opening located on the side of the shell 2 or without accommodations for receiving a needle clip on the probe, respectively. In other embodiments, the shell 2 may include a bracket for a mounting a needle clip.

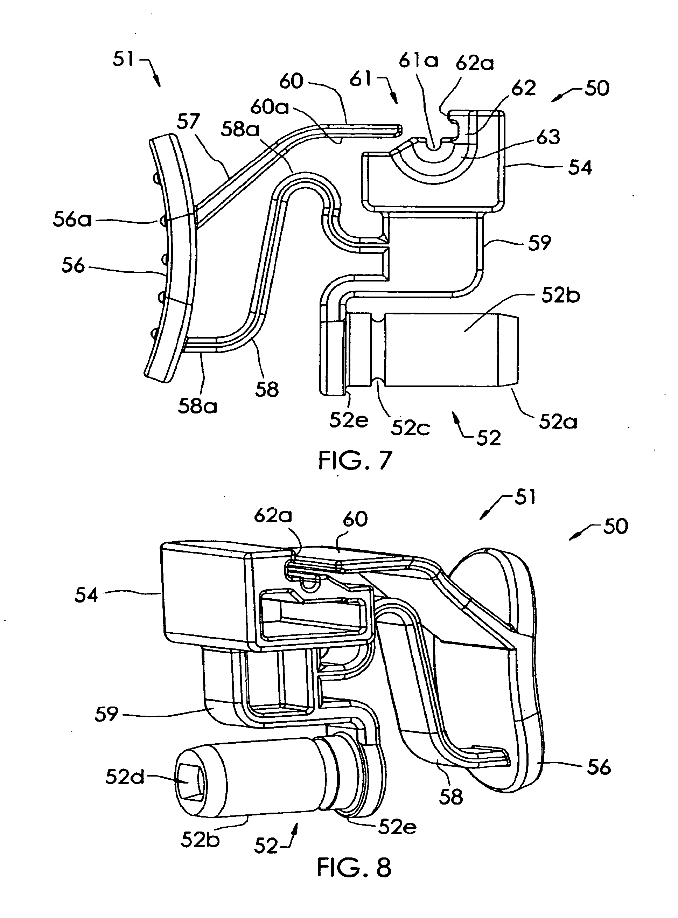

Reference will now be made to embodiments of the needle clip 50. During the course of this description, the features of the clip 50 will be described with reference to its preferred use with the probe 40. However, It will be understood that the needle clip 50 may be used with other probe types. The needle clip 50 is constructed so that the needle shaft may be separated from the probe with little unintended movement of the needle shaft while the needle shaft is embedded within the patient. FIGS. 7 and 8 illustrate two views of the needle clip 50 according to a first disclosure. The needle clip 50 may be formed as a single-piece molded part forming a holder 54 for a needle shaft and a supporting post 59 connecting the holder 54 to the connector 52. The needle holder 54 includes a semi-circular track or cradle 61 a which receives the needle shaft. An engaging portion 51 of the needle clip 50 operates to retain the needle shaft in the cradle 61 a. The engaging portion 51 includes a finger rest 56 having ridges 56a which provide a gripping feature, a flex member 58 and a fastening arm 57 having a cover 60 portion disposed adjacent to the cradle 61a.

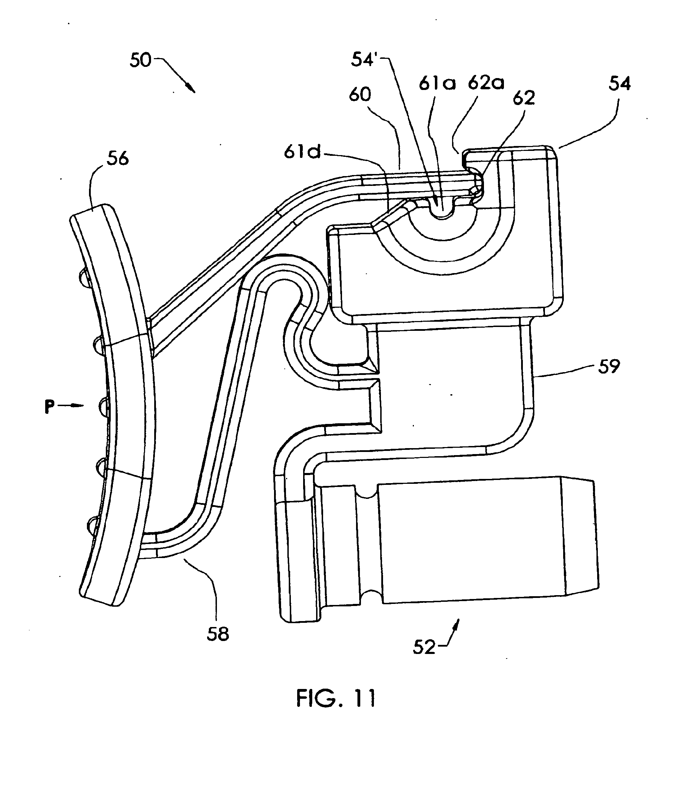

The engaging portion 51 operates in the following manner. When the needle shaft is received in the cradle 61a, the operator, i.e., a health professional, applies finger pressure to the finger rest 56 causing displacement of the cover 60 towards the right in FIG. 7 until the cover 60 is received in a notch 62 of the holder 54 (FIG. 11). Specifically, as finger pressure is applied, the cover 60 passes over a surface 61 which includes flat surfaces 61 b and 61 c on left and right sides of the cradle 61 a. A sloped surface 61 d guides the cover 60 leading edge towards the cradle 61 a (FIG. 10). The cover 60 is then received in the notch 62. A lower surface 60a of the cover 60 is now adjacent the surfaces 61b and 61c, and the portion of the surface 60a over the cradle 61a forms a closed space 54' with the cradle 61a. The closed space 54' may be thought of as a needle passageway or needle track. One or more chamfers 63 may be formed on the holder 54 to assist with guiding a tip of the needle into the cradle 61a. FIG. 11 illustrates the position of the cover 60 when finger pressure p is applied to the finger rest 56 and FIG. 7 illustrates the position of the cover 60 when there is no finger pressure applied.

The flex member 58, which may take a serpentine shape having at least one bend 58a, is attached to the finger rest 56 at a first end and to the supporting post 59 at the opposite end thereof. The flex member 58 may be replaced by a compression spring. When finger pressure is applied to the finger rest 56, the flex member 58 is elastically deformed and the cover 60 deflects into the notch 62 (FIG. 11). And when finger pressure is relieved, restoring forces in the flex member 58 withdraw the cover 60 from the notch 62 and return the cover 60 to the position shown in FIG. 7. The fastening arm 57 is sloped upwards to promote a deflection of the cover 60 into the notch 62 when finger pressure is applied. In other embodiments, the needle clip 50 may be configured so that a tension spring or a torsion spring is used in place of the illustrated flex member 58, which functions as a compression spring.

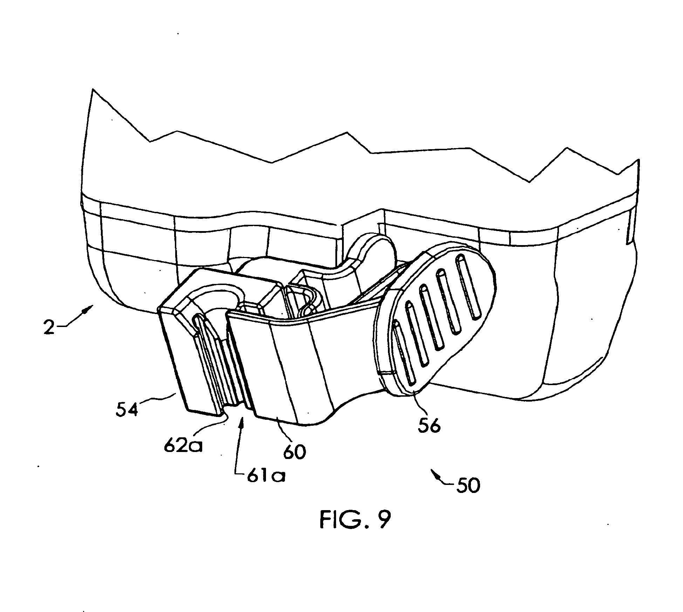

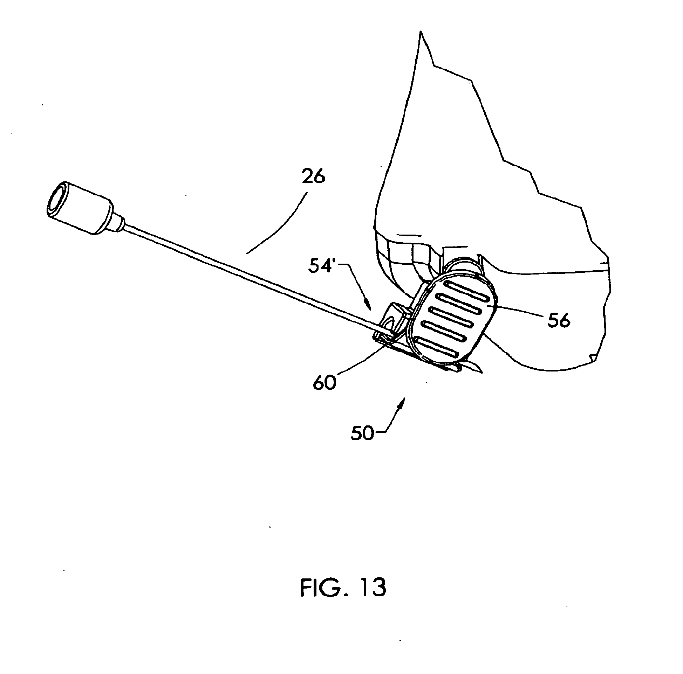

With reference to FIG. 11, a needle shaft received in the space 54' is restrained from inadvertent dislodgement from the cradle 61 a. The surface 60a and the surface of the cradle 61 a may form the closed space 54' to hold the needle shaft in place. The space 54' may be a substantially closed space, e.g., the surface 60a and surfaces on either side of the cradle 61a do not abut each other, since this closed space may also prohibit unwanted movement of the needle shaft. The space 54' is maintained only when finger pressure is applied to the finger rest 56. Hence, the needle clip 50 is devoid of a mechanical fastener or engagement between the cover 60 and the needle holder 54. When the finger pressure is released from the finger rest 56, the cover 60 naturally withdraws from the cradle 61 a by the restoring forces inherent in the compressed flex member 58. The needle clip 50 and probe 40 are thus allowed to separate from a needle shaft embedded in a patient by simply removing one's finger from the finger rest 56. A perspective view of the needle clip 50 attached to the shell 2 of the sterile probe 40, when there is no finger pressure applied to the finger rest 56, is shown in FIG. 9. FIG. 13 shows a needle shaft 26 received in the space 54' when finger pressure is applied to the finger rest 56.

The needle clip 50 may include structure that aids in preventing separation between the cover 60 and the surface 61. A separation may be caused by excessive finger pressure applied to the finger rest 56 or movement of a needle shaft which tends to force the surfaces 60a and 61 apart from each other. If a resulting space is formed between the surfaces 60a and 61, the needle shaft may become dislodged from the cradle 61a. To address these situations, a ledge 62a extending from the notch 62, may be formed adjacent and above the cradle 61a. By positioning the ledge 62a in this manner, the cover 60 leading edge will have less tendency to deflect away from the surface 61. The leading edge of the cover 60, notch 62 and/or ledge 62a may include cooperating beveled edges to assist with guiding the cover 60 into the notch 62. In alternative embodiments, the notch 62 and/or ledge 62a may be provided by a passage, hole or c-shaped member sized to receive the leading edge of the cover 60.

With reference again to FIGS. 7, 8 and 10, the connector 52 is preferably configured to connect with the shaft 49 of the probe 40. The connector 52 may be cylindrical, having an interior passage 52d which may be configured to receive a rectangular, rotatable shaft 49 of the probe 40. The shaft 49 may be a multitude of shapes, such as D-shaped, or a notched cylindrical shape, etc. such that alignment of the clip 50 to the shaft may be achieved. The exterior wall 52b of the connector 52 is formed to slide along corresponding walls of the passage 8a of the shell 2. Preferably, the connector 52 includes an annular recess 52c which receives the sealing o-ring 24. As discussed earlier, the o-ring 24 may provide a sterility barrier with the walls of the passage 8a, as well as a detent feature for the needle clip 50. When the connector 52 is fully inserted into the opening 8a and engaged with the shaft 49, an end 52e of the connector 52 abuts with the opening 8b of the shell 2. The connector 52 may include a lead-in chamfer 52a to aid with its positioning in the opening 8b. FIG. 10 illustrates a top cross-sectional view of the clip 50 received on the shaft 49.

The connector 52 may include an elastic edge or recess that snaps into a mating recess or ridge, respectively of the shaft 49 to confirm that the connector 52 is properly received on the shaft 49. The engagement may be such that an audible confirmation is provided when proper engagement is achieved, e.g., by a "clicking" sound. This confirmation may be desirable for the purpose of giving assurance that the needle shaft, when received on the cradle 61 a, is aligned with a reference axis A (see FIG. 10) of the probe's positioning and detecting device. For example, in the ultrasonic guidance system described in U.S. Application Serial No. 11/508,300, a needle tracking feature may produce a visual image of a needle's position relative to an ultrasonic image, as well as its pathway intersecting the image plane as the needle is rotated relative to the probe 40. Assuming the cradle 61 a and hence the needle shaft is positioned on the axis A, the displayed needle position reflects the true position of the needle shaft relative to the image plane.

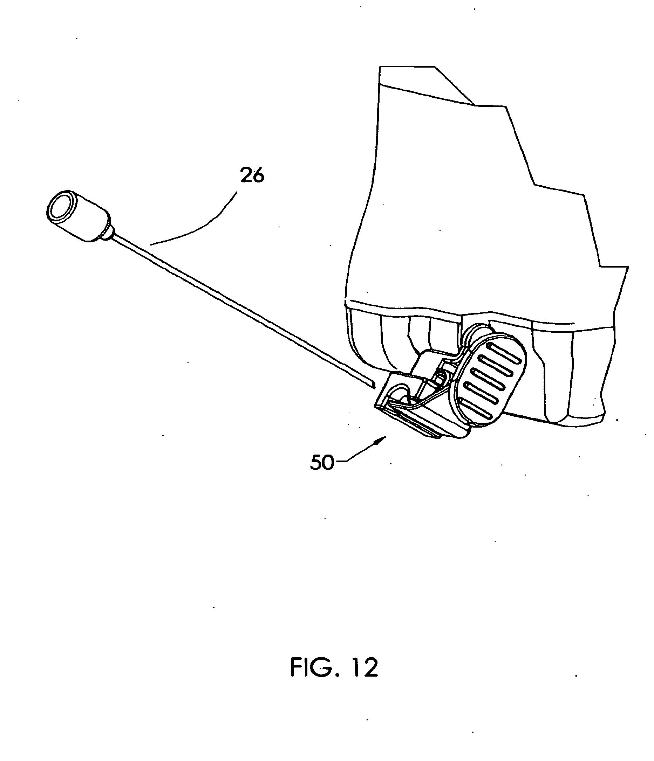

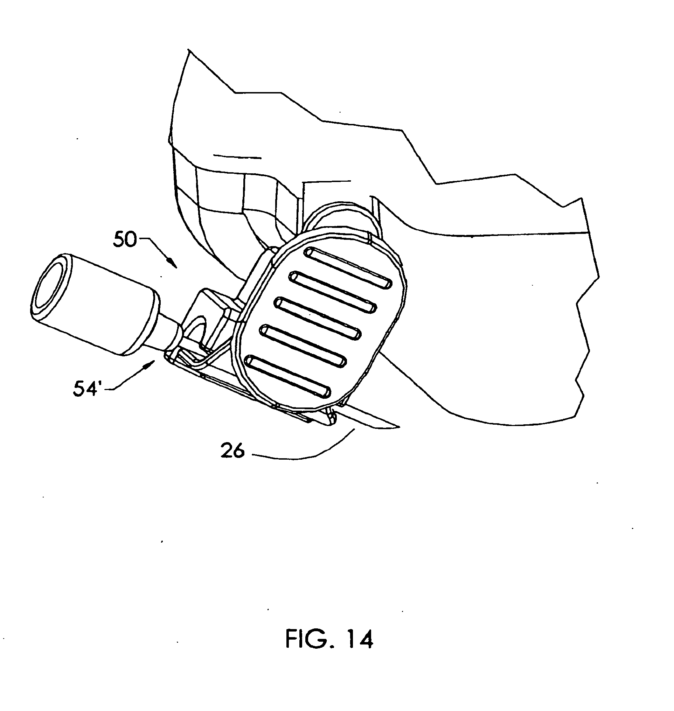

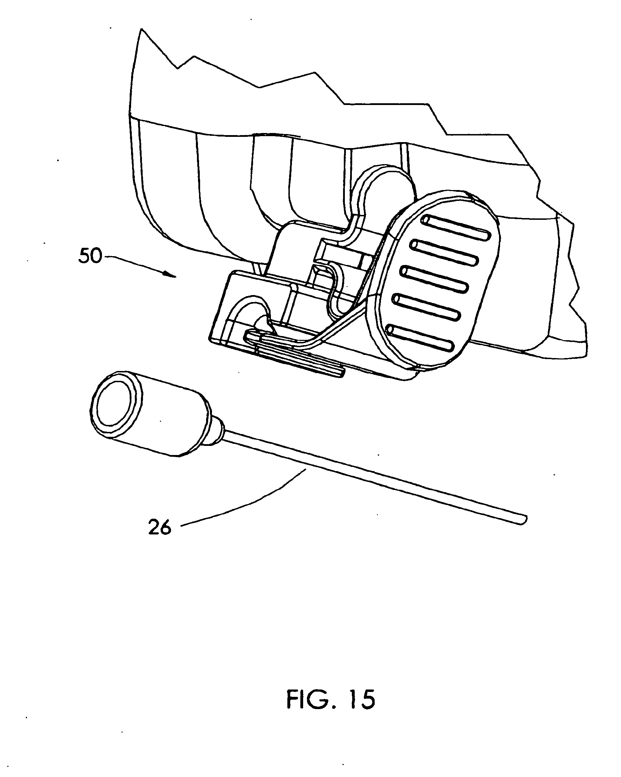

In a medical procedure, the needle clip 50 may be used to locate and insert a needle into a body of a patient using the probe 40 in the following manner. With the needle clip 50 attached to the shaft 49 of the probe 40, the health professional first locates a desired needle pathway by rotating the clip 50 relative to the probe 40. With the aid of a monitor, the movement of the needle clip and hence a needle pathway into the body may be visualized with respect to the ultrasonic image of the interior of the body, e.g., in the manner described in U.S. Application Serial No. 11/508,300. The clip 50 may be configured so that when the clip 50 is being rotated into position, the applied finger pressure also places the cover 60 over the cradle 61 a, thereby forming the closed space 54'. When the correct needle pathway is found, the needle shaft 26 is inserted into the space 54' (FIGS. 12 and 13), and then into the patient (FIG. 14). After the needle shaft 26 has been successfully located at the target, the finger pressure is removed from the needle clip 50, causing the cover to withdrawn from the cradle 61 a. The probe 40 is now easily removed from the needle shaft without additional steps needed to free the needle clip 50 from the embedded needle shaft 26 (FIG. 15).

In other embodiments, the connector 52 may be replaced by fastening structure suitable for use with other types of probes. In the case of an ultrasound imaging probe that includes a fixed-position needle clip bracket, the clip 50 may be easily configured to mount to structure provided on the exterior of a sterile shell or elsewhere, e.g., a post, sleeve, groove, etc. Additionally, the connector 52 may be configured so that it can be used with a longitudinal type ultrasound guidance system having either an adjustable or fixed needle guide feature.

## Claims

1. A needle guide, comprising:
a needle clip (50) including a needle holder (54) forming a cradle (61 a) configured to receive a needle shaft (26),
a cover (60);
a flexible member (58) having a first end connected to the needle holder (54) and a second end connected to the cover (60), wherein the cover is moveable between a first position adjacent the cradle (61 a) to a second position elastically deforming the flexible member so that the cover (60) covers the cradle (61a) to form a closed space adapted for retaining the needle shaft (26) in the cradle (61 a), **characterized in that** the cover (60) is devoid of a mechanical engagement with the cradle (61 a); and
a shell (2) configured for being coupled to an ultrasound probe (40) and including a member (8a) for receiving the needle clip (50) of the needle guide in the shell (2).

2. The needle guide of Claim 1, wherein the flexible member (58) is a beam.

3. The needle guide of Claim 2, wherein the cover (60) is connected to the holder (54) through only the beam.

4. The needle guide of Claim 1, wherein the cover (60) is a cantilever.

5. The needle guide of Claim 1, wherein the cover (60) is connected to the flexible member (58) through a finger rest (56) disposed at a second side.

6. The needle guide of Claim 1, wherein the needle guide is a one piece, molded part.

7. The needle guide of Claim 1, wherein the member is an opening (8b) and the needle clip (50) of the needle guide is receivable in the opening (8b).

8. The needle guide of Claim 1, wherein the shell (2) is a sterile shell.

9. A needle guide, comprising:
a needle holder (54) forming a surface (61) including a cradle (61a) adapted to receive a needle shaft (26) and a ledge (62a) disposed above and at a first side of the cradle (61a), and
a connector (52) coupled to the needle holder (54);
a cover (60) having an end, the cover (60) being moveable between
(i) a first position located at a second side of the cradle (61 a), and
(ii) a second position wherein the cover (60) covers the cradle (61 a) with the end between the surface (61) and the ledge (62a) so that the cover (60) and cradle (61 a) form a closed space adapted for retaining the needle shaft (26) in the cradle (61 a), the cover (60) devoid of a mechanical engagement with the cradle (61 a); and
a shell (2) configured for being coupled to an ultrasound probe (40) and including a member (8a) for receiving the connector (52) in the shell (2).

10. The needle guide of Claim 9, wherein the surface (61) includes planar surfaces formed at the first and second sides of the cradle (61 a).

11. The needle guide of Claim 9, wherein the holder (54) includes a notch (62) comprising the ledge.

12. The needle guide of Claim 11, wherein the end is received in the notch (62) when the cover (60) is in the second position.

13. The needle guide of Claim 12, wherein the end and notch (62) include beveled surfaces.

14. The needle guide of Claim 9, wherein the member (8a) is an opening (8b) and the connector (52) is rotatably receivable in the opening (8b).

## Patentansprüche

1. Nadelführung, umfassend:
- eine Nadelklammer (50), die einen Nadelhalter (54) umfasst, der eine Aufnahme (61a) bildet, die konfiguriert ist, einen Nadelschaft (26) aufzunehmen,
- eine Abdeckung (60);
- ein biegsames Element (58), das ein erstes Ende, das mit dem Nadelhalter (54) verbunden ist, und ein zweites Ende, das mit der Abdeckung (60) verbunden ist, aufweist, wobei die Abdeckung zwischen einer ersten Position neben der Aufnahme (61a) in eine zweite Position bewegbar ist, wobei es das biegsame Element elastisch verformt, so dass die Abdeckung (60) die Aufnahme (61a) abdeckt, um einen geschlossenen Raum zu bilden, der dazu geeignet ist, den Nadelschaft (26) in der Aufnahme (61a) festzuhalten,
**dadurch gekennzeichnet, dass** die Abdeckung (60) keinen mechanischen Eingriff mit der Aufnahme (61a) aufweist; und
- eine Hülle (2), die konfiguriert ist, mit einem Ultraschallkopf (40) gekoppelt zu werden, und die ein Element (8a) zur Aufnahme der Nadelklammer (50) der Nadelführung in der Hülle (2) umfasst.

2. Nadelführung nach Anspruch 1, wobei das biegsame Element (58) ein Träger ist.

3. Nadelführung nach Anspruch 2, wobei die Abdeckung (60) nur durch den Träger mit dem Halter (54) verbunden ist.

4. Nadelführung nach Anspruch 1, wobei die Abdeckung (60) ein Ausleger ist.

5. Nadelführung nach Anspruch 1, wobei die Abdeckung (60) durch eine Fingermulde (56), die auf einer zweiten Seite angeordnet ist, mit dem biegsamen Element (58) verbunden ist.

6. Nadelführung nach Anspruch 1, wobei die Nadelführung ein einstückiges Formteil ist.

7. Nadelführung nach Anspruch 1, wobei das Element eine Öffnung (8b) ist und die Nadelklammer (50) der Nadelführung in der Öffnung (8b) aufnehmbar ist.

8. Nadelführung nach Anspruch 1, wobei die Hülle (2) eine sterile Hülle ist.

9. Nadelführung, umfassend:
- einen Nadelhalter (54), der eine Oberfläche (61) bildet, die eine Aufnahme (61a), die geeignet ist, einen Nadelschaft (26) aufzunehmen, und einen Vorsprung (62a), der oberhalb und auf einer ersten Seite der Aufnahme (61a) angeordnet ist, umfasst, und
- ein Verbindungsstück (52), das mit dem Nadelhalter (54) gekoppelt ist;
- eine Abdeckung (60), die ein Ende aufweist, wobei die Abdeckung (60) bewegbar ist zwischen
(i) einer ersten Position, die sich auf einer zweiten Seite der Aufnahme (61a) befindet, und
(ii) einer zweiten Position, in der die Abdeckung (60) die Aufnahme (61a) mit dem Ende zwischen der Oberfläche (61) und dem Vorsprung (62a) derart abdeckt, dass die Abdeckung (60) und die Aufnahme (61a) einen geschlossenen Raum bilden, der geeignet ist, den Nadelschaft (26) in der Aufnahme (61a) festzuhalten, wobei die Abdeckung (60) keinen mechanischen Eingriff mit der Aufnahme (61a) aufweist; und
- eine Hülle (2), die konfiguriert ist, um mit einem Ultraschallkopf (40) gekoppelt zu werden, und die ein Element (8a) zur Aufnahme des Verbindungsstücks (52) in der Hülle (2) umfasst.

10. Nadelführung nach Anspruch 9, wobei die Oberfläche (61) ebene Oberflächen umfasst, die auf den ersten und zweiten Seiten der Aufnahme (61a) gebildet sind.

11. Nadelführung nach Anspruch 9, wobei der Halter (54) eine Kerbe (62) umfasst, die den Vorsprung umfasst.

12. Nadelführung nach Anspruch 11, wobei das Ende in der Kerbe (62) aufgenommen wird, wenn sich die Abdeckung (60) in der zweiten Position befindet.

13. Nadelführung nach Anspruch 12, wobei das Ende und die Kerbe (62) abgeschrägte Oberflächen umfassen.

14. Nadelführung nach Anspruch 9, wobei das Element (8a) eine Öffnung (8b) ist und das Verbindungsstück (52) in der Öffnung (8b) drehbar aufnehmbar ist.

## Revendications

1. Guide d'aiguille, comprenant :
une pince d'aiguille (50) incluant un porte-aiguille (54) formant un berceau (61a) configuré pour recevoir un arbre d'aiguille (26),
un couvercle (60) ;
un organe flexible (58) ayant une première extrémité raccordée au porte-aiguille (54) et une seconde extrémité raccordée au couvercle (60), où le couvercle est déplaçable entre une première position adjacente au berceau (61a) et une seconde position déformant élastiquement l'organe flexible de sorte que le couvercle (60) couvre le berceau (61a) pour former un espace fermé adapté pour retenir l'arbre d'aiguille (26) dans le berceau (61a), **caractérisé en ce que** le couvercle (60) est dépourvu d'une mise en prise mécanique avec le berceau (61a) ; et
une coque (2) configurée pour être couplée à une sonde à ultrasons (40) et incluant un organe (8a) permettant de recevoir la pince d'aiguille (50) du guide d'aiguille dans la coque (2).

2. Guide d'aiguille selon la revendication 1, dans lequel l'organe flexible (58) est une poutre.

3. Guide d'aiguille selon la revendication 2, dans lequel le couvercle (60) est raccordé au porte-aiguille (54) uniquement par l'intermédiaire de la poutre.

4. Guide d'aiguille selon la revendication 1, dans lequel le couvercle (60) est un porte-à-faux.

5. Guide d'aiguille selon la revendication 1, dans lequel le couvercle (60) est raccordé à l'organe flexible (58) par l'intermédiaire d'un repose-doigt (56) disposé au niveau d'un second côté.

6. Guide d'aiguille selon la revendication 1, dans lequel le guide d'aiguille est une pièce moulée d'un seul tenant.

7. Guide d'aiguille selon la revendication 1, dans lequel l'organe est une ouverture (8b) et la pince d'aiguille (50) du guide d'aiguille peut être reçue dans l'ouverture (8b).

8. Guide d'aiguille selon la revendication 1, dans lequel la coque (2) est une coque stérile.

9. Guide d'aiguille, comprenant :
un porte-aiguille (54) formant une surface (61) incluant un berceau (61a) adapté pour recevoir un arbre d'aiguille (26) et une pièce d'appui (62a) disposée au-dessus et au niveau d'un premier côté du berceau (61a), et
un raccord (52) couplé au porte-aiguille (54) ;
un couvercle (60) ayant une extrémité, le couvercle (60) étant déplaçable entre
(i) une première position située au niveau d'un second côté du berceau (61a), et
(ii) une seconde position dans laquelle le couvercle (60) couvre le berceau (61a) avec l'extrémité entre la surface (61) et la pièce d'appui (62a) de sorte que le couvercle (60) et le berceau (61a) forment un espace fermé adapté pour retenir l'arbre d'aiguille (26) dans le berceau (61a), le couvercle (60) étant dépourvu d'une mise en prise mécanique avec le berceau (61a) ; et
une coque (2) configurée pour être couplée à une sonde à ultrasons (40) et incluant un organe (8a) permettant de recevoir le raccord (52) dans la coque (2).

10. Guide d'aiguille selon la revendication 9, dans lequel la surface (61) inclut des surfaces planes formées au niveau des premier et second côtés du berceau (61a).

11. Guide d'aiguille selon la revendication 9, dans lequel le porte-aiguille (54) inclut une encoche (62) comprenant la pièce d'appui.

12. Guide d'aiguille selon la revendication 11, dans lequel l'extrémité est reçue dans l'encoche (62) lorsque le couvercle (60) est dans la seconde position.

13. Guide d'aiguille selon la revendication 12, dans lequel l'extrémité et l'encoche (62) incluent des surfaces biseautées.

14. Guide d'aiguille selon la revendication 9, dans lequel l'organe (8a) est une ouverture (8b) et le raccord (52) peut être reçu en rotation dans l'ouverture (8b).
